(19) Europäisches Patentamt
European Patent Office
Office européen des brevets

(11) Publication number: 0 217 577 B1

# (12) EUROPEAN PATENT SPECIFICATION

(45) Date of publication of patent specification: 10.06.92

(51) Int. Cl.5: A61K 39/395, G01N 33/531, A61K 49/02

(21) Application number: 86307031.4

(22) Date of filing: 12.09.86

## (54) Antibody complexes of hapten-modified diagnostic or therapeutic agents.

(30) Priority: 12.09.85 US 775461

(43) Date of publication of application:
08.04.87 Bulletin 87/15

(45) Publication of the grant of the patent:
10.06.92 Bulletin 92/24

(84) Designated Contracting States:
AT BE CH DE FR GB IT LI LU NL SE

(56) References cited:
US-A- 4 485 086

CHEMICAL & PHARMACEUTICAL BULLETIN, vol. 25, 1977, Tokyo - AKIRA KONO et al. : "Cobalt Chelate of Bleomycin. I. Physicochemical Properties and Distribution in Tumor Bearing Mice", pp. 1725-1731

JOURNAL OF MEDICINAL CHEMISTRY, vol. 22, no.9, September 1979, L.H. DE RIEMER et al. :"BLEDTA:Tumor Localization by a Bleomycin Analogue Containing a Metal-Chelating Group" pp 1019-1023

(73) Proprietor: HYBRITECH INCORPORATED
11095 Torreyana Road
San Diego California 92126(US)

(72) Inventor: Frincke, James M.
339 Shoemaker Lane
Solana Beach California 92075(US)
Inventor: Meyer, Damon L.
14360 Janal Way
San Diego California 92129(US)
Inventor: David, Gary S.
9477 Poole Street
La Jolla California 92037(US)
Inventor: Bartholomew, Richard M.
13143 Sundance
San Diego California 92129(US)

(74) Representative: Hudson, Christopher Mark et al
Erl Wood Manor
Windlesham Surrey GU20 6PH(GB)

EP 0 217 577 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**ANALYTICAL BIOCGEMISTRY**, vol. 142, no.1, October 1984 - C.F. MEARES et al.:"Conjugation of antibodies with Bifunctional Chelating Agents : Isothiocyanate and Bromoacetamide Reagents, Methods of Analysis, and subsequent Addition of Metal Ions", pp.68-78

**NATURE**, vol. 316, no. 6025, July 18-24, 1985 - D.T. READAN et al.:"Antibodies against metal chelates" pp. 265-267

## Description

The present invention relates generally to diagnostic and therapeutic agents. More particularly, it relates to the modification of such agents and antibody complexes therewith. In another aspect, the invention relates to processes for enhancing properties critical to the efficacy of diagnostic and therapeutic agents.

### REFERENCES

The following references are referred to herein by the corresponding number.

1. DeRiemer, L.H., et al., J. Lab. Comp. and Radiopharm. 18: 1517 (1981).
2. Meares, C.F., et al., Anal. Biochem, 142: 68 (1984).
3. Reardan, D.T., et al., Nature, 316: 265 (1985).
4. Nouel, GANN, 19: 301 (1976).
5. Kono, et al., Chem. Pharm. Bull., 25: 1725 (1977).
6. DeRiemer, L.H., et al., J. Med. Chem., 22: 1019 (1979).
7. Barranco, et al., J. Nat. Cancer Inst., 64: 913 (1980).
8. Vehara, et al., Biochem. Biophys. Res. Commun., 104: 416 (1982).
9. Wallace, R.E., et al., Current Chemotherapy and Immunotherapy, Proc 12th International Congress of Chemotherapy,: 1363 (1981).
10. James, V., et al., New Anti-Cancer Drugs: Mitoxantrone and Bisantrene, Resenzweig, et al., Ed., Raven Press, New York (1983).
11. Alberts, D.S., et al., Cancer Research, 45: 1879 (1985).
12. Gerhard, Monoclonal Antibodies, Kennett et al., Ed. Plenum Press, New York (1980)
13. Wang, et al., J. Immunol. Meth. 18: 157 (1977).
14. Parham, et al. J. Immunol. Meth. 53: 133 (1982).

Presently, a broad spectrum of diagnostic and therapeutic agents are used for in vivo diagnosis and treatment of disease, particularly cancer and infectious diseases. Diagnostic and therapeutic agents, however, often suffer from a number of limitations which inhibit their efficacy. A particular problem is that, subsequent to administration to a patient, a diagnostic or therapeutic agent may be so rapidly cleared from the body by metabolic or other pathways, or otherwise biologically inactivated, that only a relatively small percentage of the agent administered is actually effective at the target tissue. To compensate for this problem, it has been common practice to increase the dosage of the agent administered and/or prolong the period of administration and/or shorten the interval between administrations to maintain an effective concentration of the agent at the target site for a period sufficient to achieve desired results.

While the practices described above are useful, limitations are inherent in such approaches. For example, many diagnostic and therapeutic agents, such as chemotherapeutics, have toxic side effects, limiting the dosage duration or interval which may be safely used.

In certain cases, adverse side effects of promising drugs are so severe that an effective therapeutic dose cannot be safely administered. Recently, drug-antibody conjugates, comprising drugs which are covalently linked to antibodies having specificity for an in vivo target, have been developed. While such drug-antibody conjugates have been effective to increase the lifetime of certain drugs, it may be required that the drug-antibody conjugate be metabolized by a target cell for the drug to be released.

Conjugation also frequently impairs the immunoreactivity of an antibody, increases its rate of clearance, or decreases its solubility. In addition, the drug dose deliverable as an antibody conjugate may be insufficient to accomplish desired objectives. The ratio of drug to antibody, or the total drug dose deliverable, may also be limited by inherent antibody properties such as solubility and immunoreactivity.

The problems enumerated in the foregoing are not intended to be exhaustive, but rather are among many which tend to limit the potential clinical value of certain diagnostic and therapeutic agents. Accordingly, there exists a need for a more effective means by which the concentration of a diagnostic or therapeutic agent may be maintained at in vivo target sites for a period of time sufficient to attain desired results. Additionally, there exists a need for a means to concentrate a diagnostic or therapeutic agent at in vivo target sites relative to other organs and tissues. Further, there exists a need for an effective delivery system which offers flexibility and may be utilized for a broad-spectrum of diagnostic and therapeutic agents.

In accordance with the present invention, the properties critical to the efficacy of diagnostic and therapeutic agents are enhanced by forming complexes comprising such an agent, modified by a hapten to which an antibody may specifically bind, and an anti-hapten antibody selected to bind the hapten-modified agent at a site or in a manner which does not substantially impair its activity. The complexes provided by

the invention confer upon diagnostic or therapeutic agents an extended serum half-life and permit an increased concentration at *in vivo* target sites as compared with unmodified agents or hapten-modified agents.

As used, "hapten" refers to a molecule, including a small portion of an antigen, capable of specific reactivity with an antibody, but incapable of stimulating an immune response by antibody production except in combination with or as part of a carrier protein molecule. "Antibody" refers to a monoclonal antibody, polyclonal antibodies or fragments thereof, including Fab, Fab' and $F(ab')_2$ fragments or any other fragment retaining the essential binding function of an antibody, or mixtures or derivatives thereof, unless otherwise specified or required by the context in which such term is used. Additionally, in the context of the present invention, the term "activity" refers to the activity of an unmodified or hapten-modified diagnostic or therapeutic agent, or the modified activity of such an agent in conjunction with an antibody, provided such modified activity is useful for diagnostic or therapeutic purposes.

Also in accordance with the present invention, the complexes provided herein may be formed *in vitro* prior to administration to a patient, or alternatively, the hapten-modified agent and the anti-hapten antibody may be administered separately. Additionally, the anti-hapten antibody may be permitted to distribute itself throughout the patient prior to administration of the hapten-modfied agent.

As indicated above, the present invention, in one aspect, provides a complex comprising a hapten-modified diagnostic or therapeutic agent and an anti-hapten antibody selected to bind the hapten-modified agent at a site or in a manner which does not substantially impair its activity. The antibody complexation provided by the invention imparts properties which are critical to the efficacy of diagnostic and therapeutic agents modified as described herein. More specifically, the use of a hapten to modify a diagnostic or therapeutic agent, in conjunction with a suitable anti-hapten antibody, confers upon the agent such desirable properties as an extended serum half-life and an altered biodistribution which permits an increased concentration of the agent at *in vivo* target sites.

The present invention, in another aspect, provides a process for enhancing properties critical to the efficacy of a diagnostic or therapeutic agent involving the administration of such an agent, modified by a hapten prior to administration, and an anti-hapten antibody posessing the properties indicated above. In accordance with the present invention, the hapten-modified agent and the anti-hapten antibody may be combined *in vitro* and administered to a patient simultaneously. Alternatively, a suitable anti-hapten antibody preparation may be administered to a patient and, after sufficient time has elapsed to permit its distribution throughout the patient, the hapten-modified agent may be administered. The hapten-modified agent may also be administered in multiple doses over a period of time or by slow infusion subsequent to the anti-hapten antibody administration and localization. Administration of the hapten-modified agent may also involve administration of a first anti-hapten anti-tumor bifunctional antibody followed, after a period of time, by a mixture of the hapten-modified agent and a second anti-hapten antibody. The use of two anti-hapten antibodies in this manner separates the tumor localization property of the first anti-hapten antibody from the properties of the second anti-hapten antibody, namely extending serum half-life and enhancing biodistribution. In this case, a transfer occurs between the second antibody and the first antibody localized at the tumor target at areas of high first antibody concentration. Administration of the antibody complex may be accomplished by intravenous, intraperitoneal, intra-lymphatic, subcutaneous, or intramuscular injection.

In the context of the present invention, the diagnostic or therapeutic agent, hapten and anti-hapten antibody which may be suitably utilized depend upon the desired application of the invention. Accordingly, the present invention contemplates a selection of components, i.e., diagnostic or therapeutic agent, hapten and anti-hapten antibody, on the basis of the chemical and/or biological properties of each component desired for certain clinical protocols. Thus, a particular application of the invention may be optimized by the selection of components as described.

The selection of a diagnostic or therapeutic agent for use in the invention may involve the consideration of such chemical properties as water solubility, such biological properties as potency and rapid clearance from particular tissues, and physiological properties as the capability of binding with a receptor at a target site and, in certain applications, preferentially partitioning into cells in the target compartment. Among the diagnostic or therapeutic agents which are useful in the invention may be mentioned drugs such as adriamycin, mitomycin C, mitoxantrone, bleomycin, methotrexate, amphotericin B, 5-fluorouracil, actinomycin D, cis-platin, and griseofulvin; toxins such as abrin, ricin A, diphtheria toxin; proteins such as tumor necrosis factor, $\alpha$-interferon, $\beta$-interferon, nerve growth factor, prostaglandin, alkylacetyl GPC human fibrinogen, and tissue plasminogen activator; and biological response modifiers such as lymphokines, interleukin I, interleukin II and tumor necrosis factor. Drugs, and radiolabeled derivatives thereof, are preferred for use herein as diagnostic or therapeutic agents for purposes such as *in vivo* tumor imaging and therapy.

Additionally, it should be noted that the present invention is particularly advantageous for drugs which, when administered alone, present problems relating to toxicity, e.g., nephrotoxicity or toxicity associated with the blood brain barrier. The administration of an antibody complex of such a hapten-modified drug tends to maintain the hapten-modified drug in the circulatory system for a longer period of time and/or permits a more favorable biodistribution and a decreased concentration of the hapten-modified drug in critical organs and tissues other than the target tissue.

The methods by which diagnostic or therapeutic agents may be modified, i.e., derivatized, for use herein are numerous and well known to the art. For example, drugs containing nucleophilic moieties such as a primary amine, a thiol, or a hydroxyl group may be reacted with an electrophilic moiety on a hapten, such as an alkyl halide, an alkyl sulfonate, an active ester such as an N-hydroxy succinimide, or a pentafluorophenoxy carboxylic ester, an aldehyde, ketone or other electrophilic moiety such as an isothiocyano, isocyano, maleimido, or carboxylic acid chloride moiety. Conversely, drugs containing an electrophilic functional group (or a functional group that can be activated to become an electrophilic group) can be reacted with a nucleophilic moiety of a hapten. Thus, any of a wide range of functional groups may be utilized on either the selected diagnostic or therapeutic agent or the hapten, provided the groups are complementary. In accordance with the invention, diagnostic or therapeutic agents selected for use are modified such that the connecting moiety does not significantly interfere with the ability of the hapten to bind to the anti-hapten antibody to an extent that precludes advantageous combinations of the two for use herein, and the connecting moiety does not bind at a site or in a manner which substantially impairs the activity of the diagnostic or therapeutic agent. If the agent has "substantially impaired-activity" this means that the level of activity necessary for a useful diagnostic or therapeutic agent is diminished substantially or no longer present. It is important to note, however, that a decrease in activity of a diagnostic or therapeutic agent by hapten modification may not preclude its use in the invention, as an extended serum half-life and enhanced concentration at target sites may overcome a partial reduction in activity.

The selection of a hapten for use in the present invention generally depends upon whether the hapten may be readily attached to the diagnostic or therapeutic agent selected for use and, in addition, to a protein carrier which is immunogenic, i.e., a molecule which is capable of eliciting an immune response in an immunocompetent host. Further, for purposes of the present invention, the selection of a hapten may involve the consideration of such properties as water solubility, the ease of labeling with radionuclides and the ease of modification so as to affect the affinity of an anti-hapten antibody for the hapten. In certain applications, it may be desirable to modify the affinity of the anti-hapten antibody for the hapten, which may be accomplished by modifying the chemical structure of the hapten.

Suitable for use in the invention are haptens which confer desirable biological effects, such as radiosensitivity, photosensitivity and favorable kinetic properties, upon the selected diagnostic or therapeutic agent. Certain therapeutic applications which utilize radionuclides, haptens which are radiosensitizers, such as nitroaromatics, radioprotectants such as compounds containing thiol groups, or photosensitizers such as Rose Bengal, are preferably utilized. Also preferred for use for purposes of in vivo imaging and therapy are haptens which are capable of functioning as carriers of radioactive elements to enhance the delivery of radiation to a target site.

Additionally, haptens which are toxic may also be suitably utilized in certain applications of the present invention in which it is desirable to direct a toxin to an in vivo target site. For example, a toxic hapten may be utilized to modify a drug having a receptor at a target site, or a drug attached to a ligand having a receptor at a target site, in conjunction with an anti-hapten antibody.

Haptens particularly preferred for use in the invention are haptens which may be characterized as derivatives of a metal complex of benzyl EDTA. The unmodified hapten, p-nitrobenzyl EDTA, "NBE", contains structural moieties which can be conveniently converted to functional groups reactive with complementary functional groups common to a broad spectrum of diagnostic and therapeutic agents. Benzyl EDTA derivatives may also be complexed with metals, such as indium, to bind to anti-hapten antibodies, with different metal complexes exhibiting different affinities for certain anti-hapten antibodies.

Novel hapten-modified therapeutic agents particularly useful in the present invention comprise mitomycin C, a well known chemotherapeutic agent, modified by the hapten aminobenzyl EDTA isothiocyanate, or derivatives thereof. Accordingly, either metal free or metal complexes of mitomycin C aminobenzyl EDTA thiourea ("MAT") or derivatives thereof may be suitably utilized herein with an anti-hapten antibody.

Monoclonal and polyclonal antibodies are useful in the present invention as anti-hapten antibodies provided such antibodies are reactive with the hapten selected for use herein and are capable of binding the hapten-modified agent at a site or in a manner which does not substantially impair its activity. Additionally, those skilled in the art will appreciate that a single anti-hapten antibody may be utilized herein

to specifically bind to a hapten selected to modify a broad range of diagnostic and therapeutic agents.

Preferred for use in the invention are monoclonal antibodies. Monoclonal antibodies may be obtained by methods which are now well known to the art and need not be described in detail. Briefly, however, these methods generally involve the immunization of a mouse or other animal species, usually mammalian or avian, with an immunogen, i.e., the hapten conjugated to a protein carrier such as a keyhole limpet hemocyanin or thyroglobulin. Human lymphoid cells may also be obtained after immunization (natural or induced) or may be sensitized in vitro. After an immune response is generated, spleen cells of an immunized mouse having high serum titers against the hapten, or other immune lymphoid cells, are fused with cells of an established myeloma tumor line using known techniques to form hybridomas producing monoclonal antibodies. Clones of hybridomas are thereafter screened to select those which produce monoclonal antibodies having specific reactivity with the hapten of choice. Monoclonal antibodies having the desired specificity and affinity for the hapten are further screened to select those which form complexes with the hapten-modified agent which extend its serum half-life, yield favorable biodistribution characteristics, and in the case of hapten-modified drugs, result in desirable pharmacokinetics. In certain applications, it may be beneficial to use a mixture of two or more monoclonal antibodies, antibody fragments such as Fab, Fab', $F(ab')_2$ fragments or any other fragment retaining the essential binding function of an antibody or mixtures thereof, or mixtures of antibody fragments with whole antibody. Additionally, in certain applications it may be desirable to use a stoichiometric excess of antibody.

Polyclonal antibodies useful in the invention are also obtained by well known techniques. These include stimulating an immune response against the hapten selected for use in a suitable animal host such as a rabbit or other mammal. Chickens and other avian species may also be used. Serum taken from the host is then subjected to affinity purification to isolate polyclonal antibodies against the hapten. These antibodies are subsequently fractionated, if necessary, to isolate a subpopulation which is capable of complexation with the hapten-modified diagnostic therapeutic agent and possesses the properties described herein.

Particularly preferred for use in the invention are human monoclonal antibodies against the hapten, produced by hybridomas which, for example, are the product of fusion of a human B-lymphocyte with an established mammalian lymphoid line, e.g., a human or mouse myeloma line.

Also preferred for use in the present invention are chimeric monoclonal antibodies having hapten-binding specificity. As used herein, the term "chimeric antibody" refers to a monoclonal antibody comprising a variable region, i.e., binding region, and a constant region derived from different species by recombinant DNA techniques. Chimeric antibodies specific for the hapten selected for use and comprising a murine variable region and a human constant region are preferred in certain applications of the invention as such antibodies may be less immunogenic than murine monoclonal antibodies. Such murine/human chimeric antibodies are the product of immunoglobulin genes comprising DNA segments encoding for murine immunoglobulin variable regions and DNA segments encoding for human immunoglobulin constant regions. Methods for producing chimeric antibodies involve conventional recombinant DNA and gene transfection techniques well known to the art. Morrison, S.L., et al., Proc. Nat'l Acad. Sci., 81:6851 (1984).

Particularly preferred for use herein are monoclonal antibodies which are characterized in the art as hybrid or bifunctional antibodies. In accordance with the present invention, the hybrid antibodies have a dual specificity, with one or more binding sites specific for the hapten of choice and one or more binding sites specific for a target antigen, for example, an antigen associated with a tumor, an infectious organism, or other disease state. Among such antigens may be mentioned tumor-associated antigens such as carcinoembryonic antigen (CEA), prostatic acid phosphatase (PAP), prostate specific antigen (PSA), human chorionic gonadotropin (HCG), ferritin, bombesin, melanoma associated antigens p97 and gp 240. Hybrid or bifunctional antibodies for use herein may be derived either biologically, by cell fusion techniques, or chemically and may be comprised of whole antibodies aud/or fragments thereof. Methods for obtaining such hybrid antibodies are disclosed in the pending application of J. Martinis et al., Serial No. 367,784, filed April 12, 1983, [equivalent to PCT application W08303679 (October 27, 1983)] assigned to the same assignee of this application, and incorporated by reference herein.

Hybrid or bifunctional antibodies are useful in certain applications of the invention, particularly where the hapten-modified agent selected for use possesses anti-tumor activity but is either incapable of binding, or does not bind strongly, to a specific receptor at a target site. In such a case, the hybrid antibody, having specificity for the hapten-modified agent as well as the target site, will function both as a carrier of the hapten-modified agent and as a target-associated receptor for the agent. Thus, the hybrid antibody provides an effective mechanism for delivery of the hapten-modified agent to the target site.

It should be noted that complexation of the anti-hapten antibody with the hapten-modified agent, as described, may alter the inherent biological activity of the hapten-modified agent. Accordingly, the present invention contemplates selection of an anti-hapten antibody such that complexation does not diminish the

intrinsic activity of the diagnostic or therapeutic agent to an extent which is not compensated for by an extended serum half-life and enhanced delivery to a target site, as provided by the present invention.

In accordance with the invention, the complex between the anti-hapten antibody and the hapten-modified agent is in rapid equilibrium due to the non-covalent nature of the interaction between components. Significant advantages are, therefore, offered by the present invention in comparison to covalent drug-antibody conjugates. Specifically, as the anti-hapten antibody is not covalently conjugated with the hapten-modified agent, the immunoreactivity, metabolism, and solubility of the antibody will not be impaired. Further, as the diagnostic or therapeutic agent is not delivered to a target site as an antibody conjugate, the dosage of the hapten-modified agent may be increased by slow infusion over a period of time while the anti-hapten antibody remains in circulation or at the target site. By comparison, covalent drug-antibody conjugates are generally limited by the stoichiometry of the antibody conjugate and are subject to decreased immunoreactivity, increased serum clearance, and reduced solubility.

Additionally, those skilled in the art will recognize that two or more diagnostic or therapeutic agent residues may be attached, for example with a polymer, which is modified by one or more haptens in the same manner as a single agent. Accordingly, the use of such a derivatized polymer in conjunction with an anti-hapten antibody increases the dose of therapeutic or diagnostic agent deliverable to a target site by a given amount of anti-hapten antibody. Polymers useful in this regard include synthetic polymers such as ethylene-maleic anhydride, polyglutamate, polylysine, or biological polymers such as albumin or avidin.

The present invention also contemplates linkages between a hapten and a diagnostic or therapeutic agent that are labile under certain conditions. Such linkages include many that are well known to the art, such as disulfides, particularly hindered disulfides, cis-aconitic acid ester linkages, esters, N-acylaziridines, and imines. These linkages may be cleaved under any of a number of conditions, some of which may be more prevalant at the target than elsewhere. Linkages that are useful in this context are either those that would be cleaved preferentially at the target, such as cisaconitic acid linkages that are cleaved much more rapidly in a slightly acidic environment than at neutral pH, or those that are cleaved at a rate compatible with accumulation of the hapten-modified agent at the target, such as esters or N-aziridinyl amides. Such labile linkages permit the delivery of unmodified agents to the target. Thus, haptens that have desirable properties such as water solubility or ease of attachment of a radionuclide but which prevent entry of a diagnostic or therapeutic agent into target cells or otherwise inhibit the activity of the agent, are useful in the invention if connected to the diagnostic or therapeutic agent through a labile linkage as described above. Moreover, the therapeutic index of such compositions may be enhanced due to the low toxicity of the uncleaved reagent.

Further, those skilled in the art will appreciate that the present invention suggests the use of ligand-receptor complexes, other than the antibody hapten complexes provided herein, to enhance properties critical to the efficacy of diagnostic and therapeutic agents. For example, a ligand such as biotin may be used to modify a drug, with the pharmacokinetic properties of the biotin-modified drug being modified by the presence of avidin. Modification of the properties of the drug would be accomplished in the same manner that a drug-hapten is affected by an anti-hapten antibody. Similarly, an enzyme substrate or enzyme inhibitor in combination with an enzyme may be used as ligand and receptor analogously to the hapten anti-hapten antibody system provided by the invention.

The present invention is of substantial utility as it provides a means to enhance properties critical to the efficacy of diagnostic and therapeutic agents for in vivo diagnosis and therapy of disease, particularly cancer and infectious diseases. Additionally, the invention provides an effective means for the delivery of a broad-spectrum of diagnostic or therapeutic agents to in vivo targets by a single anti-hapten antibody. The present invention is further illustrated by the following non-limiting examples.

The haptens used in Examples I, II, III and IV to study the pharmacokinetic behavior of drug-haptens in the presence of an anti-hapten antibody were derivatives of benzyl EDTA. In Examples I, II and IV, the benzyl EDTA derivative used was p-bromoacetamidobenzyl EDTA, "BABE", (1,2), having a functional group which can be conveniently reacted with the functional groups of a pharmaceutical such as CL 232,468 ("Baby Blue"), a member of the mitoxantrone family of chemotherapeutic agents, and a diagnostic agent such as Co Bleomycin. In Example III, the benzyl EDTA derivative used was the indium complex of p-isothiocyanobenzyl EDTA, "ITCBE", (1,2), having a functional group which can be conveniently reacted with the functional groups of a pharmaceutical such as mitomycin C.

The pharmaceutical agents used in Examples I, II and IV were Co bleomycin and CL 232,468 ("Baby Blue"), respectively. Co bleomycin is a stable cobalt complex of the chemotherapeutic agent bleomycin, which has been found to accumulate in certain types of murine and human tumors (4,5,6) via a putative receptor (7,8) and is therefore utilized as a tumor-imaging agent using cobalt 57. However, Co-bleomycin has been shown to be rapidly excreted and lacks the chemotherapeutic potency of metal-free bleomycin.

The pharmaceutical agent used in Example II, CL 232,468, is a potent analog of the anti-tumor agent mitoxantrone (9). Mitoxantrone has been shown to rapidly clear the circulatory system and deposit in tissues, essentially fixing its biodistribution. Excretion is slow, and the dose administered is limited as a result of the toxicity of this anti-tumor agent (10, 11). The pharmaceutical agent used in Example III was mitomycin C, a chemotherapeutic agent. Mitomycin C is not commonly used as a primary treatment but in combination with other drugs due to its high toxicity and rapid metabolism in the liver.

For purposes of the present invention, the following structures and nomenclature refer to the benzyl EDTA hapten derivatives, and hapten-modified agent derivatives, used in the Examples.

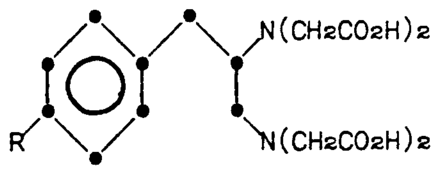

| R | Compound |
| --- | --- |
| $-NO_2$ | p-nitrobenzyl EDTA ("NBE") |
| $-NHCOCH_2Br$ | p-bromoacetamidobenzyl EDTA ("BABE") |
| -NCS | p-isothiocyanobenzyl EDTA ("ITCBE") |
| -NHCSNR' | R'-,p-aminobenzyl EDTA - thiourea where NR' = Mitomycin C ("MAT") |
| $-NHCOCH_2R'$ | R'-,p-acetamidobenzyl EDTA where R' = "Baby Blue" ("BBA") |
| $-NHCOCH_2S(CH_2)_4S$-Co-Bleomycin "BLEDTA(IV)" | |

The anti-hapten monoclonal antibody, designated CHA255, was used in Examples I, II, III and IV, demonstrating that the same anti-hapten antibody may be utilized in conjunction with different diagnostic or therapeutic agents. Additionally, the synthetic bifunctional antibody designated CHA/ZCE, was used in Examples III and IV. The CHA255 and CHA/ZCE antibodies were selected for use in the Examples only for purposes of illustration. However, alternative antibodies having specificity for the benzyl EDTA derivatives used in the Examples may be suitably utilized and interchanged with CHA255 and CHA/ZCE.

EXAMPLE I

COBALT BLEOMYCIN PREPARATION AND MODIFICATION WITH IN-BENZYL EDTA

Blenoxane® (bleomycin sulfate) was obtained from Bristol Laboratories (Syracuse, NY). A 50 ml aqueous solution of blenoxane® (4mM) and slight molar excess of $CoCl_2$ was adjusted to pH 7.0 with NaOH, and the mixture allowed to stand at room temperature overnight. The resulting mixture of bleomycin-Co(III)-$H_2O$ and bleomycin-Co(III)-$0_2H$ was acidified to pH 3.0 with HCl, and incubated for 1 day at 37° to produce substantially complete conversion of the bleomycin-Co(III)-$O_2H$ to bleomycin-Co(II)-$H_2O$ (BL-Co-$H_2O$). The solution was lyophilized and the residue was dissolved in 54 ml methanol. Undissolved material was removed by centrifugation.

Coupling 1,4-butanedithiol to BL-Co-$H_2O$.

1,4-butanedithiol was obtained from Aldrich Chemical Co. (Milwaukee, WI); BL-Co-$H_2O$ was prepared as set forth above. 6 ml of 1,4-butanedithiol was added to the solution of BL-Co-$H_2O$ in methanol and flushed with nitrogen gas. To the solution was added 1 ml triethylamine and the reaction was allowed to proceed to

completion at room temperature (3 hours). The formation of bleomycin-Co-S-$(CH_2)_4$-SH was followed by HPLC, as monitored at 254 nm. A $C_{18}$ column was used, eluted with a linear gradient from 100% Solvent A to 100% Solvent B where solvent A was an 85:15 v:v mixture of 1% ammonium acetate and acetonitrile and Solvent B was a 70:30 v:v mixture of 1% ammonium acetate and acetonitrile, run at a flow rate of 2 ml/min. If desired, the product can be isolated by preparative HPLC.

After centrifugation to remove precipitate formed during the reaction, the supernatant portion of the reaction mixture was evaporated to dryness. The dried material was dissolved in 40 ml $H_2O$ and extracted several times with ether to remove residual 1,4-butanedithiol. The aqueous phase was again evaporated to dryness and the product taken up in water.

Preparing a BL-Co-S-Butane-linked-EDTA Conjugate, BLEDTA(IV)

(S)-(p-bromoacetamidobenzyl)-EDTA (BABE) was prepared according to the procedure essentially as described in (1). BL-Co-S-$(CH_2)_4$-SH (135 mol) was comtined with a 10-fold molar excess of BABE in a total aqueous volume of 15 ml, and the solution was adjusted to pH 8.2 with NaOH. The reaction was allowed to proceed at room temperature and was monitored by HPLC, substantially as described in (1). The bleomycin-conjugate reaction product, BLEDTA(IV), was eluted immediately from the system, whereas BL-Co-S-$(CH_2)_4$-SH was retained slightly.

The conjugate was separated by ion-exchahge chromatography using an A-25 ion exchange resin obtained from Pharmacia (Piscataway, N.J.). The reaction mixture was applied to the column (1 $\times$ 45 cm) and washed with 150 ml of 0.01M ammonium formate, pH 8.2, followed by a 500 ml gradient of 0.01 M to 0.3 M ammonium formate. The column was run, and gradient steps were performed at 4° C, and product solution was monitored at 280 nm. Product was eluted at about 0.2 M ammonium formate. The pooled product fractions gave an overall product yield, as determined by absorbance at 292 nm, and based on an extinction coefficient of $2 \times 10^4$ $M^{-1}$ $cm^{-1}$, of about 20%. The product was examined by thin-layer chromatography (TLC) analysis on silica gel 60 obtained from Merck. The product showed only one spot, with $R_f$ of 0.7, when developed in 10% w/v aqueous ammonium acetate: methanol (1:1, v/v).

Proton ($^1H$) NMR spectra at 360 MHz showed two separate resonances for each of the two histidine protons in bleomycin, suggesting the existence of two separate species, perhaps involving a different arrangement of ligands around cobalt. Preliminary studies conducted in support of the invention indicate that the two species can be separated by HPLC.

Preparation of $^{111}$In-BLEDTA(IV)

Carrier free $^{111}InC1_3$ was obtained from Medi-Physics, and purified as described in (1). Fifty $\mu$1 (1-2 mCi) $InC1_3$ in 0.01 M HCl was added to an equal volume of 0.5 mM BLEDTA(IV). After vortexing, the solution was neutralized with $NaHCO_3$. The $^{111}$In-BLEDTA(IV) compound showed a single spot with an $R_f$ of 0.4 when originally prepared from pure bleomycin $A_2$. When $B_2$ is also present, another spot whose $R_f$ is 0.7 is also present.

ANTI-HAPTEN MONOCLONAL ANTIBODY PREPARATION

Antibody producing hybridoma cell lines were prepared as follows. Spleen cells from BALB/c mice immunized with the hapten described above were fused with a variant of the P3.653 myeloma cell line, (12). The resulting hybridomas were screened by a solid phase second antibody radioimmunoassay for their ability to bind $^{111}$In-aminobenzyl-EDTA (13). Based on its high titers and relatively high affinity as determined by inhibition of binding by unlabeled antigen, a monoclonal antibody designated as CHA255 was chosen for further study and injected intraperitoneally into BALB/c mice for ascites production. The monoclonal antibody was obtained and purified from mouse ascites by ion-exchange chromatography on DEAE-cellulose, essentially as described previously (14).

DETERMINATION OF IN VIVO TUMOR TARGETING.

1-2,$\mu$Ci of BLEDTA(IV)-$^{111}$In and 0-100 $\mu$g doses of CHA255 antibody were injected into six BALB/c mice. 24 hours after administration, tumor and organ uptake levels were measured. The results obtained, expressed as percent initial dose per gram tissue, are provided in Table I. The biodistribution of BLEDTA-(IV)-$^{111}$In in the absence of monoclonal antibody CHA255 is reported for comparative purposes.

The data in Table I indicate an enhanced tumor uptake of BLEDTA(IV)-$^{111}$In with the administration of

the CHA255 antibody. Further, Table I indicates an extended serum lifetime of BLEDTA(IV)-$^{111}$In on administration with the anti-hapten monoclonal antibody CHA255. Additionally, as shown by the data, the biodistribution of BLEDTA(IV)-$^{111}$In is altered. With antibody-mediated delivery, a higher percent dose of the hapten-modified pharmaceutical is concentrated at the tumor site and radiation reaching the tumor site is enhanced. At low antibody concentration, the distribution is similar to BLEDTA(IV)-$^{111}$In distribution in the absence of antibody, whereas at high antibody concentration, the distribution of the hapten-modified pharmaceutical is similar to the antibody distribution. However, as BLEDTA(IV) itself concentrates in tumor tissue by binding to a specific receptor, a monofunctional antibody need not be tumor specific to enhance localization.

TABLE I

| Organ | 0 µg CHA255 | 1 µg CHA255 | 10 µg CHA255 | 100 µg CHA255 |
|---|---|---|---|---|
| Blood | 0.0 | 0.2 | 4.3 | 16.9 |
| Bone | 0.1 | 0.0 | 0.6 | 1.8 |
| Heart | 0.1 | 0.0 | 1.0 | 4.1 |
| Kidney | 0.9 | 0.8 | 1.8 | 4.3 |
| Liver | 0.2 | 0.6 | 2.2 | 6.8 |
| Lung | 0.1 | 0.2 | 2.1 | 9.1 |
| Muscle | 0.0 | 0.0 | 0.3 | 1.3 |
| Skin | 0.0 | 0.1 | 0.7 | 1.8 |
| Spleen | 0.2 | 0.1 | 1.0 | 3.2 |
| Intestine | 0.1 | 0.1 | 0.7 | 2.0 |
| Tumor | 0.3 | 0.9 | 6.1 | 15.8 |

EXAMPLE II

MODIFICATION OF BABY BLUE WITH p-BROMOACETAMIDOBENZYL EDTA

1,4-Bis-[(2-aminoethyl)amino]-5,8-dihydroxy-9,10-anthracenedione, CL 232,468 ("Baby Blue") was obtained from American Cyanamid, Pearl River, N.Y. 3.5 mg (10 µmol) of Baby Blue were dissolved in 4 ml

dimethylacetamide (DMA) and 2 ml of an aqueous solution of 100 mM $NaHCO_3$ containing 50 mg (100 μmol) p-bromoacetamidobenzyl EDTA, "BABE", were added. The pH of the mixture was raised to 8.7 with 1 N NaOH and the mixture left stirring 24 hours.

The Baby Blue acetamidobenzyl EDTA, ("BBA") was isolated by acidification of the reaction mixture with 1 N HCl. The mixture was centrifuged and the supernatant removed. The pellet was dissolved in water with the addition of IN NaOH. The precipitation - centrifugation cycle was repeated three times. The final pellet was dissoved in water and thereafter precipitated with acetone and isolated by centrifugation. The acetone precipitation - centrifugation was repeated three times. The final solution, containing a minimum of sodium hydroxide, was lyophilized. The yield from a typical preparation was about 40%, based on an extinction coefficient of about $1.6 \times 10^4$ 1 $M^{-1}$ $cm^{-1}$ at the absorption maximum near 620 nm.

DETERMINATION OF BIODISTRIBUTION

Pharmacokinetic experiments on BBA-$^{111}$In were performed by intravenous injection of 0 or 100 μl of a saline solution containing the anti-hapten antibody CHA255, prepared as described in Example I into six mice. Carrier free BBA-$^{111}$In, prepared by mixing a solution of $^{111}InCl_3$ with a citrate solution and the resulting solution with Img BBA, and diluting with saline to obtain a concentration of 10 μCi/100 μl, was injected into each mouse 96 hours after the antibody. After allowing BBA $^{111}$In to circulate for specific durations of time, each mouse was sacrificed, dissected, and the fraction of the injected radiation in each organ determined. The results obtained, expressed as percent initial dose per organ, are provided in Tables II and III.

**TABLE II**

**% Injected Dose of BBA per Organ in the Absence of CHA255**

| | 1 hr | 4 hr | 24 hr |
|---|---|---|---|
| Blood | 4.4 | 3.0 | 0.2 |
| Bone | 6.9 | 6.8 | 4.1 |
| Heart | 0.2 | 0.2 | 0.0 |
| Kidney | 1.1 | 1.0 | 1.0 |
| Liver | 2.8 | 2.9 | 2.0 |
| Lung | 0.4 | 0.4 | 0.0 |
| Muscle | 5.5 | 5.6 | 0.8 |
| Skin | 9.2 | 9.8 | 1.4 |
| Spleen | 0.2 | 0.2 | 0.1 |
| Intestine | 5.6 | 5.2 | 1.4 |
| Urine | - | - | 53.7 |
| Feces | - | - | 10.6 |

## TABLE III

% Injected Dose of BBA-111 In per Organ in the Presence of Various Levels of CHA255

| Time (h) | 1 | | | 4 | | | 24 | | | 48 | | | 72 | | |
|---|---|---|---|---|---|---|---|---|---|---|---|---|---|---|---|
| CHA255 (µg) | 2 | 20 | 200 | 2 | 20 | 200 | 2 | 20 | 200 | 2 | 20 | 200 | 2 | 20 | 200 |
| Blood | 37.5 | 29.9 | 39.2 | 27.8 | 27.8 | 24.3 | 5.2 | 15.8 | 15.5 | 1.6 | 11.9 | 16.0 | - | 8.6 | 12.6 |
| Bone | 9.6 | 4.2 | 4.0 | 4.1 | 4.0 | 3.6 | 3.6 | 3.4 | 3.6 | 3.0 | 3.1 | 3.6 | - | 2.0 | 4.2 |
| Heart | 0.7 | 0.4 | 0.4 | 0.5 | 0.5 | 0.3 | 0.1 | 0.4 | 0.3 | 0.0 | 0.2 | 0.4 | - | 0.2 | 0.3 |
| Kidney | 0.9 | 1.1 | 1.0 | 0.7 | 0.8 | 0.6 | 0.7 | 0.6 | 0.5 | 0.5 | 0.5 | 0.5 | - | 0.5 | 0.6 |
| Liver | 10.6 | 42.4 | 43.3 | 7.8 | 40.1 | 51.3 | 5.1 | 25.1 | 41.5 | 3.2 | 18.6 | 38.4 | - | 16.1 | 33.9 |
| Lung | 1.5 | 1.3 | 1.4 | 1.0 | 1.2 | 0.9 | 0.3 | 1.2 | 0.8 | 0.2 | 0.7 | 1.1 | - | 0.4 | 0.8 |
| Muscle | 15.0 | 5.4 | 4.2 | 8.3 | 5.2 | 3.8 | 3.9 | 7.2 | 8.5 | 1.3 | 8.9 | 7.3 | - | 6.4 | 9.0 |
| Skin | 11.1 | 2.9 | 1.4 | 6.3 | 4.1 | 2.0 | 2.6 | 7.5 | 7.8 | 0.9 | 6.1 | 9.6 | - | 4.2 | 9.2 |
| Spleen | 0.5 | 0.9 | 0.9 | 0.5 | 0.6 | 0.6 | 0.2 | 0.4 | 0.5 | 0.1 | 0.4 | 0.6. | - | 0.3 | 0.7 |
| Intestine | 5.9 | 6.7 | 3.6 | 6.5 | 7.0 | 4.0 | 1.9 | 6.0 | 3.3 | 0.9 | 4.9 | 3.9 | - | 3.7 | 4.0 |
| Urine | - | - | - | - | - | - | 43.7 | 13.3 | 4.2 | 51.6 | 20.2 | 6.1 | - | 20.9 | 9.8 |
| Feces | - | - | - | - | - | - | 11.4 | 8.5 | 4.3 | 15.1 | 17.0 | 6.6 | - | 19.9 | 10.5 |

For purposes of comparison, 2-200 µg of CHA255 were administered to six mice and allowed to circulate 96 hours. 10 µCi $^{111}$In-nitrobenzyl EDTA, "NBE", (hapten) were then injected into each mouse. After 1 hour the animals were sacrificed and dissected, and the fraction of the injected radiation in radiation in each organ determined. The results obtained, expressed as percent initial dose per organ, are provided in Table IV.

## TABLE IV

$^{111}$In-Nitrobenzyl EDTA Concentration 1 h Post Injection of Hapten for Anti-Indium EDTA Antibody (CHA255) in Various Compartments in the Mouse

| Antibody Dose | 200 μg | 20 μg | 2 μg | Hapten Control |
|---|---|---|---|---|
| Compartment | % Dose per organ | % Dose per organ | % Dose per organ | % Dose per organ @ 1 hour |
| Blood | 41.1% | 34.4% | 24.6% | 1.1% |
| Bone | 6.4 | 4.2 | 4.9 | 2.0 |
| Heart | 0.8 | 0.8 | 0.4 | 0.03 |
| Kidney | 1.1 | 1.3 | 0.8 | 0.4 |
| Liver | 11.7 | 11.0 | 5.8 | 1.1 |
| Lung | 2.3 | 1.7 | 1.8 | 0.2 |
| Muscle | 10.1 | 13.7 | 12.5 | 2.1 |
| Skin | 6.7 | 7.7 | 10.0 | 2.2 |
| Spleen | 0.5 | 0.5 | 0.3 | 0.04 |
| Intestine* | 8.1 | 7.9 | 8.8 | 11.6 |

*Includes feces

To illustrate the biodistribution of $^{111}$In labeled CHA255, the anti-hapten antibody was conjugated with DTPA and labeled with $^{111}$In. Six mice per time point were injected with 100 μl of an antibody solution containing 10 μCi and sacrificed at the indicated times. The results obtained, expressed as percent initial dose per organ, are provided in Table V. (The urine level at 24 hours reflects $^{111}$In that was not atached to the antibody. It has been shown in numerous previous experiments that labeled antibodies show urine levels

of about 5% of the dose at 24 hours.)

TABLE V

% Injected Dose per Organ of $^{111}$In-CHA255

| Time (h) | 4 | 24 | 48 | 72 |
|---|---|---|---|---|
| Blood | 39.6 | 20.6 | 13.0 | 8.9 |
| Bone | 4.0 | 4.0 | 5.0 | 4.7 |
| Heart | 0.4 | 0.4 | 0.3 | 0.2 |
| Kidney | 1.6 | 1.7 | 1.4 | 1.1 |
| Liver | 9.3 | 10.1 | 8.6 | 8.2 |
| Lung | 1.4 | 1.5 | 0.8 | 0.7 |
| Muscle | 10.4 | 7.9 | 8.6 | 7.0 |
| Skin | 7.5 | 6.8 | 7.3 | 5.1 |
| Spleen | 0.5 | 0.5 | 0.6 | 0.9 |
| Intestine | 4.3 | 4.5 | 4.6 | 4.2 |
| Urine | - | 20.0 | 23.0 | 31.0 |
| Feces | - | 4.6 | 8.4 | 15.0 |

To emphasize the change in biodistribution due to the presence of antibody, six nephrectomized mice were treated in a manner identical to that in the experiment described in Tables II and III. The mice were sacrificed 4 hours after injection of the BBA-$^{111}$In. The results obtained are provided in Table VI.

TABLE VI

% Injected Dose per Organ 4 Hours After Injection of BBA-$^{111}$In

| | Normal Animals | Nephrectomicze Animals | Normal Animals With CHA255 |
|---|---|---|---|
| Blood | 3.0 | 13.4 | 24.3 |
| Bone | 6.8 | 19.4 | 3.6 |
| Heart | 0.2 | 0.3 | 0.3 |
| Kidney | 1.0 | 0.0 | 0.6 |
| Liver | 2.9 | 6.1 | 51.3 |
| Lung | 0.4 | 1.1 | 0.9 |
| Muscle | 5.6 | 20.6 | 3.8 |
| Skin | 9.8 | 29.5 | 2.0 |
| Spleen | 0.2 | 0.4 | 0.6 |
| Intestine | 5.2 | 12.1 | 4.0 |

As shown by Table II, hapten-modified Baby Blue ("BBA") has a different biodistribution than unmodified Baby Blue. Additionally, Table II indicates that elimination of BBA from the body occurs rapidly through the kidneys and urine, and tissue penetration is negligible. Table III demonstrates distribution of the hapten-modified pharmaceutical in the presence of CHA255 as a function of time. At 2 $\mu$g, CHA255 primarily raises the blood level at the early timepoints, and some organ levels are also elevated. At 20 $\mu$g, and 200 $\mu$g CHA255 organ levels are dramatically elevated, especially the liver. However, the excretion rate is significantly reduced by the antibody, and the excretory route is altered to about 50% liver. The distribution and pharmacokinetics observed with this drug-hapten/anti-hapten antibody system are entirely different from the distribution of the hapten in the presence of the antibody (Table IV). Thus, the distribution of the complex between the hapten-modified pharmaceutical and the anti-hapten antibody behaves as a function of antibody concentration.

The distribution of labeled BBA is compared in normal animals, nephrectomized animals, and animals

treated with anti-hapten antibody in Table VI. In comparing organ levels of normal animals with nephrectomized animals, the relative amounts in each organ are consistent. A comparison of the 6 organs having the highest percentage of the drug-hapten, as measured by the presence of the label, indicates that the organ levels are substantially the same. The removal of an excretory route, raises the concentration in each organ but does not change the relative ratios in the organs. In contrast, 20 µg CHA255 causes a radical shift in organ distribution of the drug-hapten, implying that the antibody not only extends the half-life of the drug hapten and increases its concentration in tissues, but also changes its biodistribution pattern.

EXAMPLE III

PREPARATION OF MITOMYCIN C MODIFIED BY AMINOBENZYL EDTA THIOUREA ("MAT")

MAT was prepared separately as the indium loaded derivative, MAT-In, and the metal-free compound, MAT-0, so that concentration and specific activity could be varied independently in various experiments.

MAT-In

To 2.8 ml of 48.6 mM ITCBE ($1.36 \times 10^{-4}$ mol) in 0.3 M HCl, 1.04 ml of $1.32 \times 10^{-1}$ M $InCl_3$ solution ($1.37 \times 10^{-1}$ mol) was added and the solution was stirred minutes. The pH of the solution was then raised to pH 9 with triethylamine. Mitomycin C (Sigma Chemical Co., St. Louis, MO), 20 mg ($5.4 \times 10^{-5}$ mol), was dissolved in 3.5 ml dimethylacetamide (DMA) and added over 5 minutes to the stirred ITCBE- solution.

After 52 hours of stirring at room temperature the reaction mixture was diluted to 1 L with water and loaded onto a DEAE sephadex A25 anion exchange column in the formate form. The product was eluted with a linear gradient of ammonium formate, pH 7, from 20 to 200 mM, and the fractions analyzed by ultraviolet absorbance at 362 nm and 250 nm. Those fractions that had an absorbance at 362 nm above 1.0 and an $A_{250}/A_{362}$ ratio around 0.75 (ratio should be constant for all pure product fractions) were pooled, frozen and lyophilized.

Pure MAT-In was taken up in water, analyzed by UV absorbance spectrum and HPLC profile, and stored frozen. The HPLC profile was observed using a Hewlett-Packard 100 × 2.1 mm HyperSil ODS column under the following conditions:

| | |
|---|---|
| Flow Rate = | 0.4 mL/min |
| Gradient = | 100% A - 100% B in 10 minutes |
| A = | 50 mM triethylammonium acetate 10 Mm EDTA, pH 6 |
| B = | methanol |

Impurities resulting from hydrolysis of the MMC moiety ("mitocene" derivative) were retained longer: 6.32 minutes for indium loaded material and 5.83 minutes for metal-free, and were distinguishable by their altered spectra (observed with a diode array detector) which has a peak around 310 nm, no peak at 362 nm, and increased absorbance at 280 nm. Chelate by-products, if present, appeared with a retention time of about 3.90 minutes.

MAT-0

Synthesis of MAT-0 was similar to MAT-In, but with the dium omitted from the procedure. Thus, the pH of the ITCBE solution was raised to pH 9 with triethylamine, and MMC in DMA was added. After 52 hours, the reaction mixture was diluted in 20 mM ammonium formate instead of water for application to the ion exchange column. The column gradient was 200 to 500 mM ammonium formate instead of 20 to 200 mM.

In addition to the analysis performed on MAT-In, MAT-0 was titrated with indium for chelate capacity. The structure of MAT-0 is shown below:

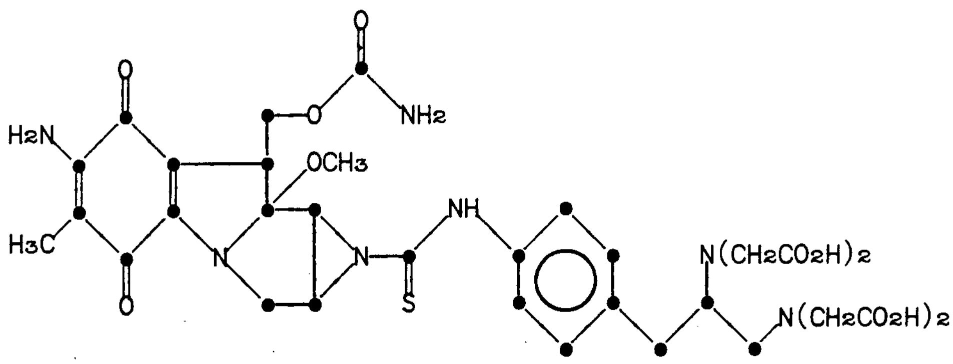

Experiments were performed to determine the biodistribution of MAT-In at various times after injections in the absence and presence of monoclonal antibody CHA255. MAT-0 was mixed with $^{111}$In in the presence of ammonium citrate at pH 6.5. A TLC (Silica gel eluted with 1:1 10% ammonium acetate:methanol) showed that 100% of the $^{111}$In was bound by the MAT-0. The MAT-$^{111}$In so formed was mixed with an appropriate amount of MAT-In, prepared as described above, to bring the MAT-In concentration to the level required for the experiment.

PREPARATION OF BIFUNCTIONAL ANTIBODY CHA/ZCE

Purified monoclonal antibody CHA255 and monoclonal antibody ZCE025 were digested with pepsin to produce F(ab')$_2$ fragments. The reaction mixture was purified by gel filtration prior to reduction to Fab fragments. After reduction, Fab' fragments were purified by gel filtration. Bifunctional antibody CHA/ZCE was prepared through recombination of two Fab' fragments using protocols which regenerate the disulfide linkage or, alternatively, join the two fragments through a bis-maleimide bridge. Reformation of a disulfide-linked bifunctional antibody was achieved through dithionitrobenzoic acid ("DTNB") activation of one Fab' fragment, purification and reaction with the second Fab' fragment, in 25% yield. An alternative disulfide stabilized linkage system was developed using bis-maleimide methylether (BMME). By substituting BMME for DTNB in the above protocol, a recombined bifunctional antibody was obtained in 50% yield.

Synthetic bifunctional antibody titers are similar to those of a biologically produced bifunctional antibody with the same specificities.

DETERMINATION OF BIODISTRIBUTION AND TUMOR LOCALIZATION

Table VII presents the results of three experiments in which $1.25 \times 10^{-10}$ mol MAT-In (containing approximately 10 $\mu$Ci $^{111}$In) was injected into Balb/c mice that had been injected with 0, $1.25 \times 10^{-10}$ or $1.25 \times 10^{-9}$ mol CHA255 48 hours previously. After the time indicated the mice were dissected and the organs counted. The data are reported as the percent of injected dose in the indicated organ divided by the weight of the organ, except for urine and feces, for which percent dose is reported, and the resultant values have been normalized to 100% recovery of $^{111}$In. (Actual $^{111}$In recoveries in these experiments ranged from 70 to 95%).

**TABLE VII**

**MAT-In**
**NORMALIZED % DOSE/GRAM**

| Time | 1 Hr | | | 4 Hr | | | 24 Hr | | | 48 Hr | | | 72 Hr | | |
|---|---|---|---|---|---|---|---|---|---|---|---|---|---|---|---|
| µg CHA255 | 0 | 20 | 200 | 0 | 20 | 200 | 0 | 20 | 200 | 0 | 20 | 200 | 0 | 20 | 200 |
| Blood | 1.4 | 9.4 | 31.9 | 1.0 | 7.2 | 23.7 | 0.4 | 4.2 | 16.4 | - | 2.8 | 14.7 | - | 1.6 | 11.2 |
| Bone | 1.4 | 2.1 | 3.5 | 0.9 | 1.4 | 2.2 | 0.9 | 1.7 | 2.5 | - | 1.0 | 1.6 | - | 1.3 | 1.3 |
| Heart | 0.7 | 2.2 | 7.3 | 0.5 | 1.9 | 5.6 | 0.4 | 1.3 | 3.6 | - | 0.8 | 3.0 | - | 0.8 | 2.6 |
| Kidney | 6.9 | 7.7 | 7.3 | 5.4 | 6.2 | 5.1 | 4.4 | 6.3 | 4.1 | - | 4.8 | 4.1 | - | 3.8 | 3.6 |
| Liver | 1.9 | 6.7 | 17.0 | 1.6 | 5.5 | 15.4 | 1.3 | 9.4 | 19.7 | - | 8.2 | 20.4 | - | 10.4 | 18.4 |
| Lung | 1.1 | 3.7 | 12.7 | 0.8 | 3.0 | 8.7 | 0.5 | 2.7 | 6.6 | - | 1.6 | 6.3 | - | 1.5 | 5.7 |
| Muscle | 0.4 | 0.8 | 1.2 | 0.3 | 0.5 | 0.8 | 0.2 | 0.4 | 1.1 | - | 0.3 | 1.0 | - | 0.3 | 0.8 |
| Skin | 1.6 | 2.2 | 0.9 | 0.8 | 1.2 | 1.4 | 0.5 | 1.0 | 2.6 | - | 0.7 | 2.0 | - | 0.6 | 1.7 |
| Spleen | 0.6 | 1.8 | 6.0 | 0.5 | 1.4 | 4.3 | 0.5 | 1.7 | 4.0 | - | 1.2 | 3.8 | - | 1.6 | 3.5 |
| Intestine | 6.2 | 6.2 | 2.4 | 6.0 | 6.9 | 3.1 | 0.4 | 1.1 | 2.0 | - | 0.6 | 1.6 | - | 0.4 | 1.0 |
| Urine* | 20.0 | 3.2 | 0.6 | 13.5 | 15.6 | 1.5 | 28.0 | 20.5 | 3.3 | - | 23.9 | 4.2 | - | 22.6 | 5.4 |
| Feces* | 3.5 | 0.4 | 0.0 | 6.8 | 4.3 | 0.1 | 23.5 | 19.8 | 10.8 | - | 33.7 | 10.2 | - | 70.7 | 33.1 |

*: % Dose

Table VII demonstrates that CHA255 changes the relative organ distribution of the reagent and increases the time that the reagent stays in circulation, in a dose dependent manner.

Table VIII demonstrates results of three experiments in which MAT-In was mixed with the synthetic bifunctional antibody CHA/ZCE (BFA) in 1:1, 1:2, and 1:5 mole ratios and injected into nude mice carrying a T380 cell line tumor. The bifunctional antibody dose was 14 µg per mouse in each case. The data are represented as Tumor/Organ ratios in Table IX. The optimal differential between tumor an other organs occurs between 48 and 72 hours. These tumor organ concentration ratios compare favorably with the ratios for traditional chemotherapeutic agents.

# TABLE VIII

MAT-In
NORMALIZED % DOSE/GRAM

| Time | 1 Hr | | | 4 Hr | | | 24 Hr | | | 48 Hr | | | 72 Hr | | |
|---|---|---|---|---|---|---|---|---|---|---|---|---|---|---|---|
| MAT/BFA | 1:1 | 1:2 | 1:5 | 1:1 | 1:2 | 1:5 | 1:1 | 1:2 | 1:5 | 1:1 | 1:2 | 1:5 | 1:1 | 1:2 | 1:5 |
| Blood | 25.1 | - | - | 13.3 | 15.4 | 18.3 | 4.9 | 5.7 | 6.4 | 1.7 | 2.8 | 2.9 | - | 2.6 | 2.2 |
| Bone | 2.6 | - | - | 1.6 | 1.7 | 1.9 | 1.1 | 1.6 | 1.5 | 0.6 | 1.4 | 0.8 | - | 1.6 | 1.2 |
| Heart | 6.3 | - | - | 4.8 | 4.6 | 6.5 | 2.1 | 3.0 | 2.8 | 1.1 | 1.9 | 1.7 | - | 2.3 | 1.8 |
| Kidney | 20.8 | - | - | 24.0 | 30.3 | 39.3 | 21.5 | 32.4 | 32.9 | 12.0 | 23.1 | 22.4 | - | 24.9 | 21.7 |
| Liver | 5.1 | - | - | 4.5 | 5.7 | 5.8 | 4.0 | 6.6 | 5.8 | 2.9 | 5.6 | 5.0 | - | 9.3 | 6.3 |
| Lung | 6.4 | - | - | 3.9 | 4.9 | 5.1 | 2.0 | 2.4 | 2.9 | 1.1 | 1.7 | 1.6 | - | 2.2 | 1.7 |
| Muscle | 1.2 | - | - | 0.8 | 1.0 | 1.1 | 0.6 | 0.9 | 1.0 | 0.4 | 0.7 | 0.6 | - | 0.7 | 0.7 |
| Skin | 2.5 | - | - | 3.1 | 2.9 | 3.2 | 2.8 | 4.2 | 4.4 | 2.0 | 3.4 | 3.9 | - | 4.6 | 4.1 |
| Spleen | 3.9 | - | - | 2.6 | 3.0 | 3.8 | 2.1 | 2.8 | 3.3 | 1.4 | 3.1 | 2.7 | - | 10.2 | 3.6 |
| Tumor | 4.6 | - | - | 6.6 | 4.6 | 7.6 | 7.1 | 11.7 | 15.6 | 5.7 | 11.4 | 13.7 | - | 11.0 | 14.4 |
| Intestine | 2.5 | - | - | 4.2 | 4.9 | 3.4 | 1.8 | 2.8 | 2.3 | 0.8 | 1.8 | 1.0 | - | 3.7 | 1.4 |
| Urine* | 2.5 | - | - | 2.9 | 1.0 | 2.9 | 11.4 | 8.3 | 16.4 | 24.3 | 13.6 | 16.2 | - | 13.6 | 17.6 |
| Feces* | 0.0 | - | - | 0.5 | 0.6 | 1.7 | 20.9 | 15.8 | 16.4 | 28.1 | 25.4 | 30.5 | - | 25.5 | 31.8 |

*: % Dose

**TABLE IX**

**MAT-In**

**(NORMALIZED % DOSE/GRAM IN TUMOR)/(NORMALIZED % DOSE/GRAM IN ORGAN)**

| Time | 1 Hr | | | 4 Hr | | | 24 Hr | | | 48 Hr | | | 72 Hr | | |
|---|---|---|---|---|---|---|---|---|---|---|---|---|---|---|---|
| MAT/BFA | 1:1 | 1:2 | 1:5 | 1:1 | 1:2 | 1:5 | 1:1 | 1:2 | 1:5 | 1:1 | 1:2 | 1:5 | 1:1 | 1:2 | 1:5 |
| Blood | 0.2 | - | - | 0.5 | 0.3 | 0.4 | 1.5 | 2.0 | 2.4 | 3.4 | 4.0 | 4.6 | - | 4.2 | 6.5 |
| Bone | 1.8 | - | - | 4.1 | 2.8 | 4.0 | 6.7 | 7.4 | 10.7 | 8.8 | 8.2 | 16.8 | - | 6.8 | 11.7 |
| Heart | 0.7 | - | - | 1.4 | 1.0 | 1.3 | 3.4 | 3.8 | 5.5 | 5.3 | 5.9 | 7.9 | - | 4.8 | 7.8 |
| Kidney | 0.2 | - | - | 0.3 | 0.2 | 0.2 | 0.3 | 0.4 | 0.5 | 0.5 | 0.5 | 0.6 | - | 0.4 | 0.7 |
| Liver | 0.9 | - | - | 1.5 | 0.8 | 1.3 | 1.8 | 1.8 | 2.7 | 1.9 | 2.0 | 2.8 | - | 1.2 | 2.3 |
| Lung | 0.7 | - | - | 1.7 | 0.9 | 1.5 | 3.6 | 4.8 | 5.4 | 5.4 | 6.5 | 8.6 | - | 4.9 | 8.4 |
| Muscle | 3.7 | - | - | 7.8 | 4.7 | 6.9 | 11.1 | 13.3 | 15.6 | 13.6 | 16.7 | 22.0 | - | 15.9 | 19.5 |
| Skin | 1.9 | - | - | 2.2 | 1.6 | 2.4 | 2.5 | 2.8 | 3.5 | 2.8 | 3.4 | 3.5 | - | 2.4 | 3.5 |
| Spleen | 1.2 | - | - | 2.5 | 1.5 | 2.0 | 3.4 | 4.1 | 4.8 | 3.9 | 3.6 | 5.0 | - | 1.1 | 4.0 |
| Intestine | 1.9 | - | - | 1.6 | 0.9 | 2.2 | 4.0 | 4.2 | 6.9 | 7.3 | 6.3 | 13.1 | - | 3.0 | 10.6 |

*: % Dose

## EXAMPLE IV

This example demonstrates that the invention permits the separation of two functions that the anti-hapten antibody performs, namely biodistribution and localization. Because of a by-product in the preparation of the $F(ab')_2$ bifunctional antibody CHA/ZCE used in Example III, covalent attachment to a radiotracer showed a localization of approximately 30% in the kidneys, a property that is undesirable for the biodistribution of therapeutic agents. Yet localization of this particular fragment in tumor targets is rapid and otherwise favorable.

Table X shows the results of a study in which 14 μg of the CHA/ZCE fragment was injected, and allowed to localize to a tumor target for 24 hours. A mixture of 1 μg CHA255 with carrier free BLEDTA (IV)-In was then injected and the organ distribution measured at the indicated intervals. The data are compared with protocols in which (columns 1 and 4) carrier free BLEDTA (IV)-In was mixed with 20 μg CHA255 (i.e., no tumor specificity), and (columns 2 and 5) in which 28 μg CHA/ZCE was used to deliver and localize the drug hapten. Comparison of the tumor levels in column 3 with 1 and column 6 with 4 clearly shows that hapten has transerred from the carrier CHA255 to the localized CHA/ZCE. Furthermore, comparison of column 3 with 2 and column 6 with 5 indicates that the transfer protocol gives a more favorable organ distribution, especially in terms of kidney concentration.

TABLE X

BLEDTA (IV)·In HAPTEN TRANSFER
NORMALIZED % DOSE/GRAM

| Time | 4 Hr | | | 24 Hr | | |
|---|---|---|---|---|---|---|
| Protocol | BLEDTA(IV) In CHA255 | BLEDTA(IV) In CHA/ZCE | BLEDTA(IV) In CHA255→CHA/ZCE | BLEDTA(IV) In CHA255 | BLEDTA(IV) In CHA/ZCE | BLEDTA(IV) In CHA255→CHA/ZCE |
| Blood | 25.2 | 15.2 | 9.4 | 9.5 | 2.5 | 1.2 |
| Bone | 2.2 | 1.9 | - | 1.1 | 1.1 | - |
| Heart | 5.1 | 6.3 | - | 2.9 | 2.0 | - |
| Kidney | 4.8 | 52.1 | 11.7 | 3.0 | 43.7 | 6.5 |
| Liver | 5.1 | 6.9 | 3.8 | 3.1 | 5.6 | 2.0 |
| Lung | 10.5 | 4.8 | 2.9 | 4.3 | 1.6 | 0.7 |
| Muscle | 1.3 | 1.1 | - | 0.6 | 0.6 | - |
| Skin | 3.5 | 3.6 | - | 4.0 | 3.2 | - |
| Spleen | 3.6 | 3.6 | 2.1 | 2.9 | 2.8 | 0.9 |
| Tumor | 3.8 | 9.1 | 15.0 | 4.0 | 8.8 | 6.1 |
| Intestine | 1.7 | 1.4 | 2.4 | 1.6 | 0.8 | 1.5 |
| Urine* | 0 | 6.1 | 62.8 | 25.6 | 44.1 | 73.0 |
| Feces* | 0 | 0.1 | 1.6 | 5.9 | 6.5 | 14.6 |

*: % Dose

## Claims

**Claims for the following Contracting States : BE, CH, DE, FR, GB, IT, LI, LU, NL, SE**

**1.** A composition comprising a hapten-modified diagnostic or therapeutic agent and an anti-hapten

antibody, said anti-hapten antibody being selected to bind said hapten-modified agent in a manner which does not substantially impair the activity of said agent.

2. A composition as claimed in claim 1 in which said anti-hapten antibody is a monoclonal antibody or fragment thereof.

3. A composition as claimed in claim 2 wherein said anti-hapten antibody comprises an antibody fragment selected from the group consisting of Fab, Fab', $F(ab')_2$ fragments or an antibody fragment retaining the essential binding function of said antibody.

4. A composition as claimed in claim 2 in which said anti-hapten antibody is a chimeric antibody or hybrid monoclonal antibody having a dual specificity, one of which is directed against said hapten-modified agent and the other against a target antigen.

5. A composition as claimed in claim 4 in which said target antigen is a tumor-associated antigen and said hapten-modified agent has anti-tumor activity.

6. A composition as claimed in claim 5 in which said target antigen is selected from the group consisting of carcinoembryonic antigen, prostatic acid phosphatase, prostate specific antigen, human choriconic gonadotropin ferritin, bombesin, and melanoma-associated antigens p97 and gp240.

7. A composition as claimed in claim 1 wherein said hapten-modified agent is selected from the group consisting of drugs, toxins, proteins, and biological response modifiers or radiolabeled derivatives thereof.

8. A composition as claimed in claim 7 in which said hapten-modified agent is a drug capable of binding to a receptor at said target site.

9. A composition as claimed in any of claims 1 to 8 in which said hapten-modified agent and said anti-hapten antibody are complexed in vitro.

10. A product containing a hapten-modified diagnostic or therapeutic agent and an anti-hapten antibody, as claimed in any one of claims 1 to 8 as a combined preparation for simultaneous or sequential therapeutic or diagnostic use.

11. A composition as claimed in any one of claims 1 to 8 for therapeutic or diagnostic use.

12. A composition as claimed in claim 1 in which said hapten-modified agent comprises mitomycin C aminobenzyl EDTA thiourea ("MAT"), a metal complex or a derivative thereof.

13. Hapten-modified mitomycin C.

14. Mitomycin C amino-benzyl EDTA thiourea, a metal complex or derivatives thereof.

15. A pharmaceutical formulation which comprises a hapten-modified diagnostic or therapeutic agent, as defined in any one of claims 1 to 8 or 12 and an anti-hapten antibody, as defined in any one of claims 1 to 8, associated with a pharmaceutically-acceptable carrier, diluent or vehicle therefor.

**Claims for the following Contracting State : AT,**

1. A process for preparing a pharmaceutical composition which comprises complexing a hapten-modified diagnostic or therapeutic agent with an anti-hapten antibody, said anti-hapten antibody being selected to bind said hapten-modified agent in a manner which does not substantially impair the activity of said agent.

2. A process for preparing a composition as claimed in claim 1 in which said anti-hapten antibody is a monoclonal antibody or fragment thereof.

3. A process for preparing a composition as claimed in claim 2 wherein said anti-hapten antibody comprises an antibody fragment selected from the group consisting of Fab, Fab', $F(ab')_2$ fragments or an antibody fragment retaining the essential binding function of said antibody.

4. A process for preparing a composition as claimed in claim 2 in which said anti-hapten antibody is a chimeric antibody or hybrid monoclonal antibody having a dual specificity, one of which is directed against said hapten-modified agent and the other against a target antigen.

5. A process for preparing a composition as claimed in claim 4 in which said target antigen is a tumor-associated antigen and said hapten-modified agent has anti-tumor activity.

6. A process for preparing a composition as claimed in claim 5 in which said target antigen is selected from the group consisting of carcinoembryonic antigen, prostatic acid phosphatase, prostate specific antigen, human chorionic gonadotropin ferritin, bombesin, and melanoma-associated antigens p97 and gp240.

7. A process for preparing a composition as claimed in claim 1 wherein said hapten-modified agent is selected from the group consisting of drugs, toxins, proteins, and biological response modifiers or radio-labeled derivatives thereof.

8. A process for preparing a composition as claimed in claim 7 in which said hapten-modified agent is a drug capable of binding to a receptor at said target site.

9. A process for preparing a composition as claimed in any of claims 1 to 8 in which said hapten-modified agent and said anti-hapten antibody are complexed in vitro.

10. A process for preparing a composition as claimed in claim 1 in which said hapten-modified agent comprises mitomycin C aminobenzyl EDTA thiourea ("MAT"), a metal complex or a derivative thereof.

**Revendications**
**Revendications pour les Etats contractants suivants : BE, CH, DE, FR, GB, IT, LI, LU, NL, SE**

1. Composition comprenant un agent thérapeutique ou diagnostique modifié par un haptène et un anticorps anti-haptène, ledit anticorps anti-haptène étant choisi pour se lier avec ledit agent modifié par un haptène, d'une manière qui n'entrave pas essentiellement l'activité dudit agent.

2. Composition selon la revendication 1, dans laquelle ledit anticorps anti-haptène est un anticorps monoclonal ou un fragment de ce dernier.

3. Composition selon la revendication 2, dans laquelle ledit anticorps anti-haptène comprend un fragment d'anticorps choisi parmi le groupe comprenant des fragments Fab, Fab', $F(ab')_2$ ou encore un fragment d'anticorps qui garde la fonction essentielle de liaison dudit anticorps.

4. Composition selon la revendication 2, dans laquelle ledit anticorps anti-haptène est un anticorps chimérique ou un anticorps monoclonal hybride possédant une double spécificité, dont l'une est dirigée contre ledit agent modifié par un haptène et l'autre contre un antigène cible.

5. Composition selon la revendication 4, dans laquelle ledit antigène cible est un antigène associé à une tumeur et ledit agent modifié par un haptène possède une activité anti-tumorale.

6. Composition selon la revendication 5, dans laquelle ledit antigène cible est choisi parmi le groupe comprenant l'antigène carcino-embryonnaire, la phosphatase de l'acide prostatique, l'antigène spécifique à la prostate, la gonadotrophine chorionique humaine, la ferritine, la bombésine, ainsi que les antigènes p97 et gp240 associes à un mélanome.

7. Composition selon la revendication 1, dans laquelle ledit agent modifié par un haptène est choisi parmi le groupe comprenant des médicaments, des toxines, des protéines et des modificateurs de réponse biologique ou encore des dérivés radiomarqués de ces derniers.

8. Composition selon la revendication 7, dans laquelle ledit agent modifié par un haptène est un médicament capable de se lier à un récepteur audit site cible.

9. Composition selon l'une quelconque des revendications 1 à 8, dans laquelle ledit agent modifié par un haptène et ledit anticorps anti-haptène sont complexés in vitro.

10. Produit contenant un agent diagnostique ou thérapeutique modifié par un haptène et un anticorps anti-haptène, selon l'une quelconque des revendications 1 à 8, comme préparation combinée pour une utilisation thérapeutique ou diagnostique simultanée ou successive.

11. Composition selon l'une quelconque des revendications 1 à 8, destinée à une utilisation thérapeutique ou diagnostique.

12. Composition selon la revendication 1, dans laquelle ledit agent modifié par un haptène comprend la mitomycine C-EDTA aminobenzylé-thio-urée ("MAT"), un de ses complexes métalliques ou encore un de ses dérivés.

13. Mitomycine C modifiée par un haptène.

14. Mitomycine C-EDTA aminobenzylé-thio-urée, un de ses complexes métalliques ou un de ses dérivés.

15. Formulation pharmaceutique qui comprend un agent diagnostique ou thérapeutique modifié par un haptène, tel que défini dans l'une quelconque des revendications 1 à 8 ou 12, ainsi qu'un anticorps anti-haptène tel que défini dans l'une quelconque des revendications 1 à 8, en association avec un support, un diluant ou un véhicule pharmaceutiquement acceptable pour l'agent et l'anticorps.

**Revendications pour l'Etat contractant suivant : AT**

1. Procédé pour préparer une composition pharmaceutique, qui consiste à complexer un agent thérapeutique ou diagnostique modifié par un haptène, avec un anticorps anti-haptène, ledit anticorps anti-haptène étant choisi pour se lier avec ledit agent modifié par un haptène, d'une manière qui n'entrave pas essentiellement l'activité dudit agent.

2. Procédé pour préparer une composition selon la revendication 1, dans lequel ledit anticorps anti-haptène est un anticorps monoclonal ou un fragment de ce dernier.

3. Procédé pour préparer une composition selon la revendication 2, dans lequel ledit anticorps anti-haptène comprend un fragment d'anticorps choisi parmi le groupe comprenant des fragments Fab, Fab', $F(ab')_2$ ou un fragment d'anticorps qui garde la fonction essentielle de liaison dudit anticorps.

4. Procédé pour préparer une composition selon la revendication 2, dans lequel ledit anticorps anti-haptène est un anticorps chimérique ou un anticorps monoclonal hybride possédant une double spécificité, dont l'une est dirigée contre ledit agent modifié par un haptène et l'autre contre un antigène cible.

5. Procédé pour préparer une Composition selon la revendication 4, dans lequel ledit antigène cible est un antigène associé à une tumeur et ledit agent modifié par un haptène possède une activité anti-tumorale.

6. Procédé pour préparer une composition selon la revendication 5, dans lequel ledit antigène cible est choisi parmi le groupe comprenant l'antigène carcino-embryonnaire, la phosphatase de l'acide prostatique, l'antigène spécifique à la prostate, la gonadotrophine chorionique humaine, la ferritine, la bombésine, ainsi que les antigènes p97 et gp240 associés à un mélanome.

7. Procédé pour préparer une composition selon la revendication 1, dans lequel ledit agent modifié par un haptène est choisi parmi le groupe comprenant des médicaments, des toxines, des protéines et des modificateurs de réponse biologique ou encore des dérivés radiomarqués de ces derniers.

8. Procédé pour préparer une composition selon la revendication 7, dans lequel ledit agent modifié par un haptène est un médicament capable de se lier à un récepteur audit site cible.

9. Procédé pour préparer une composition selon l'une quelconque des revendications 1 à 8, dans lequel ledit agent modifié par un haptène et ledit anticorps anti-haptène sont complexés in vitro.

10. Procédé pour préparer une composition selon la revendication 1, dans lequel ledit agent modifié par un haptène comprend la mitomycine C-EDTA aminobenzylé-thio-urée ("MAT"), un de ses complexes métalliques ou encore un de ses dérivés.

**Patentansprüche**
**Patentansprüche für folgende Vertragsstaaten : BE, CH, DE, FR, GB, IT, LI, LU, NL, SE**

1. Zusammensetzung enthaltend ein haptenmodifiziertes diagnostisches oder therapeutisches Mittel und einen Antihapten-Antikörper, wobei der Antihapten-Antikörper so ausgewählt ist, daß er das haptenmodifizierte Mittel in einer solchen Weise bindet, daß die Aktivität des Mittels nicht wesentlich beeinträchtigt wird.

2. Zusammensetzung nach Anspruch 1, worin der Antihapten-Antikörper ein monoklonaler Antikörper oder ein Fragment davon ist.

3. Zusammensetzung nach Anspruch 2, worin der Antihapten-Antikörper ein Antikörper-Fragment, ausgewählt aus der Gruppe bestehend aus Fab, Fab', $F(ab')_2$-Fragmenten oder einem Antikörper-Fragment, bei dem die wesentliche Bindungsfunktion des Antikörpers aufrechterhalten ist, umfaßt.

4. Zusammensetzung nach Anspruch 2, worin der Antihapten-Antikörper ein chimerer Antikörper oder ein monoklonaler Hybrid-Antikörper mit einer zweifachen Spezifität ist, wovon eine Spezifität gegen das haptenmodifizierte Mittel gerichtet ist und die andere gegen ein Ziel-Antigen.

5. Zusammensetzung nach Anspruch 4, worin das Ziel-Antigen ein tumorassoziiertes Antigen ist und das haptenmodifizierte Mittel Antitumoraktivität hat.

6. Zusammensetzung nach Anspruch 5, worin das Ziel-Antigen ausgewählt ist aus der Gruppe bestehend aus karzinoembryonalem Antigen, saurer Prostata-Phosphatase, Prostata-spezifischem Antigen, Humanchoriongonadotropin, Ferritin, Bombesin und den melanomassoziierten Antigenen p97 und gp240.

7. Zusammensetzung nach Anspruch 1, worin das haptenmodifizierte Mittel ausgewählt ist aus der Gruppe bestehend aus Arzneimitteln, Toxinen, Proteinen und Modifikatoren der biologischen Antwort oder radioaktiv markierten Derivaten davon.

8. Zusammensetzung nach Anspruch 7, worin das haptenmodifizierte Mittel ein Arzneimittel ist, das einen Rezeptor an der Zielstelle binden kann.

9. Zusammensetzung nach einem der Ansprüche 1 bis 8, worin das haptenmodifizierte Mittel und der Antihapten-Antikörper in vitro komplexiert werden.

10. Produkt enthaltend ein haptenmodifiziertes diagnostisches oder therapeutisches Mittel und einen Antihapten-Antikörper nach einem der Ansprüche 1 bis 8 als kombiniertes Präparat für die gleichzeitige oder aufeinanderfolgende therapeutische oder diagnostische Verwendung.

11. Zusammensetzung nach einem der Ansprüche 1 bis 8 zur therapeutischen oder diagnostischen Verwendung.

12. Zusammensetzung nach Anspruch 1, worin das haptenmodifizierte Mittel Mitomycin C-Aminobenzyl-EDTA-Thioharnstoff ("MAT"), einen Metallkomplex oder ein Derivat davon umfaßt.

13. Haptenmodifiziertes Mitomycin C.

14. Mitomycin C-Aminobenzyl-EDTA-Thioharnstoff, Metallkomplex oder Derivat davon.

15. Pharmazeutisches Präparat, umfassend ein haptenmodifiziertes diagnostisches oder therapeutisches Mittel nach einem der Ansprüche 1 bis 8 oder 12 und einen Antihapten-Antikörper nach einem der Ansprüche 1 bis 8 zusammen mit einem pharmazeutisch annehmbaren Träger, Verdünnungsmittel oder Hilfsstoff dafür.

**Patentansprüche für folgenden Vertragsstaat : AT**

1. Verfahren zur Herstellung einer pharmazeutischen Zusammensetzung, umfassend, daß man ein haptenmodifiziertes diagnostisches oder therapeutisches Mittel mit einem Antihapten-Antikörper komplexiert, wobei der Antihapten-Antikörper so ausgewählt ist, daß er das haptenmodifizierte Mittel in einer solchen Weise bindet, daß die Aktivität des Mittels nicht wesentlich beeinträchtigt wird.

2. Verfahren zur Herstellung einer Zusammensetzung nach Anspruch 1, worin der Antihapten-Antikörper ein monoklonaler Antikörper oder ein Fragment davon ist.

3. Verfahren zur Herstellung einer Zuammensetzung nach Anspruch 2, worin der Antihapten-Antikörper ein Antikörper-Fragment umfaßt, ausgewählt aus der Gruppe bestehend aus Fab, Fab', $F(ab')_2$-Fragmenten oder einem Antikörper-Fragment, das die wesentliche Bindungsfunktion des Antikörpers aufrechterhält.

4. Verfahren zur Herstellung einer Zusammensetzung nach Anspruch 2, worin der Antihapten-Antikörper ein chimerer Antikörper oder ein monoklonaler Hybrid-Antikörper mit einer zweifachen Spezifität ist, wovon eine Spezifität gegen das haptenmodifizierte Mittel gerichtet ist und die andere gegen ein Ziel-Antigen.

5. Verfahren zur Herstellung einer Zusammensetzung nach Anspruch 4, worin das Ziel-Antigen ein tumorassoziiertes Antigen ist und das haptenmodifizierte Mittel Antitumoraktivität hat.

6. Verfahren zur Herstellung einer Zusammensetzung nach Anspruch 5, worin das Ziel-Antigen ausgewählt ist aus der Gruppe bestehend aus Karzinoembryonalem Antigen, saurer Prostata-Phosphatase, Prostata-spezifischem Antigen, Humanchoriongonadotropin, Ferritin, Bombesin und den melanomassoziierten Antigenen p97 und gp240.

7. Verfahren zur Herstellung einer Zusammensetzung nach Anspruch 1, worin das haptenmodifizierte Mittel ausgewählt ist aus der Gruppe bestehend aus Arzneimitteln, Toxinen, Proteinen und Modifikatoren der biologischen Antwort oder radioaktiv markierten Derivaten davon.

8. Verfahren zur Herstellung einer Zusammensetzung nach Anspruch 7, worin das haptenmodifizierte Mittel ein Arzneimittel ist, das an einen Rezeptor an der Zielstelle binden kann.

9. Verfahren zur Herstellung einer Zusammensetzung nach einem der Ansprüche 1 bis 8, worin das haptenmodifizierte Mittel und der Antihapten-Antikörper in vitro komplexiert werden.

10. Verfahren zur Herstellung einer Zusammensetzung nach Anspruch 1, worin das haptenmodifizierte Mittel Mitomycin C-Aminobenzyl-EDTA-Thioharnstoff ("MAT"), einen Metallkomplex oder ein Derivat davon umfaßt.